# EUROPEAN PATENT APPLICATION

(11) **EP 4 400 581 A1**
(43) Date of publication of application: **17.07.2024**
(21) Application number: 22867704.3
(22) Date of filing: 07.09.2022
(51) Int. Cl.: C12N 5/0789, A01K 67/027

(54) **METHOD FOR PREPARING PLURIPOTENT STEM CELL-DERIVED HEMATOPOIETIC STEM CELL AND METHOD FOR CONSTRUCTING HUMANIZED MOUSE MODEL BY USING HEMATOPOIETIC STEM CELL THUS PREPARED**

(30) Priority: 10.09.2021 KR 20210121174
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: KANG, Eun Ju, Seoul 04595 (KR); LEE, Yeonmi, Seoul 05270 (KR)
(74) Representative: Weickmann & Weickmann PartmbB
(86) International application number: PCT/KR2022/013463
(87) International publication number: WO 2023/038436

(57) **Abstract**

Provided is a method for preparing pluripotent stem cell-derived hematopoietic stem cells and a method of constructing a humanized mouse model using the prepared hematopoietic stem cells. According to an aspect, the method of preparing hematopoietic stem cells identified an optimal differentiation condition according to a combination of low-molecular-weight compounds and protein growth factors without gene insertion, and thus may differentiate hematopoietic stem cells from pluripotent stem cells at high yield.

## Description

### Technical Field

The present disclosure relates to a method of preparing pluripotent stem cell-derived hematopoietic stem cells and a method of constructing a humanized mouse model by using the prepared hematopoietic stem cells.

### Background Art

Because research on diseases in humans has limitations, disease models using animals known to be genetically very similar to humans are being usefully used. Specifically, the target disease is developed in a model animal, and various treatments are applied to explore treatment methods. However, it is unknown whether a treatment that has a therapeutic effect in an animal disease model will have the same effect when applied to humans. Therefore, a treatment confirmed in an animal disease model cannot be directly applied to humans and requires several steps before clinical application.

In order to use animal disease models more effectively, efforts have been recently made to build humanized animal models with immune systems similar to those of humans. To build a humanized animal model, especially a humanized mouse, a method of transplanting hematopoietic stem cells into a mouse with deficient immune function has been used. For example, when human CD34⁺ cells were transplanted into a SCID (severe combined immunodeficiency) mouse, it was confirmed that human-derived hematopoietic stem cells were expressed in small amounts and were systematically remodeled in all tissues of the mouse.

Hematopoietic stem cells are derived from umbilical cord blood, exist mainly in bone marrow, can produce blood cells such as red blood cells, white blood cells and platelets through proliferation and differentiation, and have the characteristics of stem cells including self-renewal capacity, high capacity for cell division, and multi-potential differentiation capacity. Hematopoietic stem cells may produce about 4 × 10¹¹ blood cells per day in a stable state. Such hematopoietic stem cells may be used to construct a humanized animal model, and furthermore, hematopoietic stem cell transplantation therapy is clinically active in the treatment of blood cancer-related diseases including acute leukemia, chronic leukemia, aplastic anemia, myelodysplastic syndrome and multiple myeloma, solid tumors including breast cancer, kidney cancer and ovarian cancer, and autoimmune diseases including refractory systemic lupus erythematosus and refractory rheumatoid arthritis.

However, because these hematopoietic stem cells exist at very low proportions, there are many difficulties in isolating them, and the differentiation and proliferation conditions are complex and difficult, and therefore the development of technology to promote effective differentiation and self-proliferation of hematopoietic stem cells has not yet been achieved.

Accordingly, the present inventors solved the above problems by developing a method that may easily obtain hematopoietic stem cells by highly efficient differentiation from pluripotent stem cells using only an optimal combination of low-molecular-weight compounds and protein growth factors without gene insertion.

### Disclosure

### Technical Problem

An aspect provides a method of preparing hemaopoietic stem cells, including (A) primary culturing pluripotent stem cells (PSCs) in a medium including a glycogen synthase kinase (GSK) inhibitor to obtain mesodermal cells, (B) secondary culturing the mesodermal cells to induce or differentiate the mesodermal cells into hemangioblasts, (C) thirdly culturing the hemangioblasts to obtain Endothelial-to-Hematopoietic Transition (EHT)-induced hemangioblasts, and (D) fourthly culturing the EHT-induced hemangioblasts to induce or differentiate the EHT-induced hemangioblasts into hematopoietic stem cells.

Another aspect provides a method of constructing a humanized animal model, including transplanting or injecting hematopoietic stem cell prepared by the method of preparing hematopoietic stem cells into an individual other than a human.

Another aspect provides a humanized animal model prepared by the method of constructing a humanized animal model.

### Technical Solution

An aspect is to provide a method of preparing hemaopoietic stem cells, including (A) primary culturing pluripotent stem cells (PSCs) in a medium including a glycogen synthase kinase (GSK) inhibitor to obtain mesodermal cells, (B) secondary culturing the mesodermal cells to induce or differentiate the mesodermal cells into hemangioblasts, (C) thirdly culturing the hemangioblasts to obtain Endothelial-to-Hematopoietic Transition (EHT)-induced hemangioblasts, and (D) fourthly culturing the EHT-induced hemangioblasts to induce or differentiate the EHT-induced hemangioblasts into hematopoietic stem cells.

As used herein, the term "hematopoietic stem cell (HSC)" refers to a progenitor cell of an undifferentiated bone marrow hematopoietic cell that produces red blood cells, white blood cells, and platelets, and is also called a hemocytoblast. In the bone marrow blood of normal people, about 1 % of cells (CD34-positive cells) have the ability to produce all blood cells, and these are called hematopoietic stem cells. They are the mother cells that produce blood and are found throughout the body, but are especially produced in large quantities in the bone marrow. From these cells, red blood cells, white blood cells, and platelets, which correspond to the cells that make up blood, are differentiated and produced. In addition, they have a self-replication capacity that allows them to yield identical copies of themselves, and they account for about 0.05 % to 0.25 % of the total hematopoietic stem cells in the bone marrow. Peripheral blood hematopoietic stem cells are derived from the bone marrow and are hematopoietic stem cells that circulate in the bloodstream and have the properties of self-replication and differentiation into mature cells.

As used herein, the term "pluripotent stem cell (PSC)" refers to a stem cell that may differentiate into almost all types of cells constituting the endoderm, mesoderm, and ectoderm. The pluripotent stem cells are capable of almost permanent or long-term cell proliferation while maintaining an undifferentiated state through in vitro culture, display a normal chromosomal pattern and, under appropriate conditions, have the ability to differentiate into all cells of the three germ layers (ectoderm, mesoderm, and endoderm). Traditionally, embryonic stem cells obtained through embryonic tissues and blastocysts derived from fertilized eggs are included as representative pluripotent stem cells. However, embryonic stem cells have a possibility of immune rejection due to differences in histocompatibility antigens between donor and host, and thus genetically customized pluripotent stem cells have been developed to overcome this problem. One of these is cloned embryonic stem cells obtained from an embryo after replacing the nucleus of a somatic cell and an egg, and the other is induced pluripotent stem cells (iPS) that are pluripotent stem cells almost identical to embryonic stem cells, obtained from reprogramming somatic cells through gene manipulation. In particular, the induced pluripotent stem cells (iPS) make it possible to obtain customized pluripotent stem cells from the patient's own somatic cells without the bioethical conflicts that existed with embryonic stem cells or cloned embryonic stem cells in the past.

The pluripotent stem cells may be selected from a group including embryonic stem cells isolated from early embryos, induced pluripotent stem cells or similar cells, and embryonic germ cells isolated from primordial germ cells in the fetal period, and may be human pluripotent stem cells.

The method of preparing hemaopoietic stem cells may be a method of preparing pluripotent stem cell-derived hematopoietic stem cells, and specifically, a method of inducing/differentiating pluripotent stem cells into hematopoietic stem cells or enhancing differentiation from pluripotent stem cells into hematopoietic stem cells. Additionally, the hematopoietic stem cells prepared by the method may be CD34 positive (CD34⁺) cells, specifically may be CD34 positive and CD45 positive (CD34+ CD45+) cells.

The term "differentiation" as used herein refers to a process by which non-specialized cells develop into specific cells, and particularly includes a process of developing from stem cells into specific cells. In the present disclosure, pluripotent stem cells were used as cells with differentiation potential, and the pluripotent stem cells may differentiate into hematopoietic stem cells via mesoderm (mesodermal cells) and hemangioblasts.

In the method, (A) may be of primary culturing a pluripotent stem cell, specifically may be of inducing or differentiating pluripotent stem cells into mesoderm (mesodermal cells).

The primary culturing may be of culturing pluripotent stem cells in a medium including a glycogen synthase kinase (GSK) inhibitor, and the medium may be a mesodermal differentiation/induction medium.

As used herein, the term "glycogen synthase kinase (GSK) inhibitor" refers to substances that target GSK1/2, an upstream molecule of GSK1/2 involved in the glycogen synthase kinase (GSK) signaling process. The GSK inhibitors may include one or more selected from the group consisting of CHIR99021, 1-azakenpaullone, AZD2858, BIO, ARA014418, Indirubin-3'-monoxime, 5-lodo-indirubin-3'-monoxime, kenpaullone, SB-415286, SB-216763, Maybridge SEW00923SC, (Z) -5-(2,3-Methylenedioxyphenyl)-imidazolidine-2,4-dione, TWS 119, CHIR98014, SB415286, Tideglusib, LY2090314, and pharmaceutically acceptable salts thereof, and specifically may be CHIR99021 or a pharmaceutically acceptable salt thereof.

As used herein, the term "CHIR99021 (6-[[2-[[4-(2,4-Dichlorophenyl)-5-(5-methyl-1H-imidazol-2-yl)-2-pyrimidinyl]amino]ethyl]amino]-3-pyridinecarbonitrile)" is a glycogen synthase kinase (GSK) inhibitor that targets GSK1/2, an upstream molecule of GSK1/2 involved in the GSK signaling process, and may be expressed as aminopyrimidine. The CHIR99021 is represented by the chemical formula C₂₂H₁₈Cl₂N₈ and may be expressed by the structural formula of Formula 1 below.

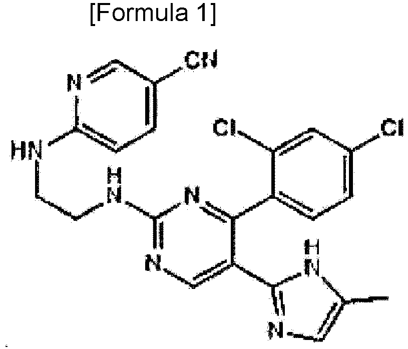

The CHIR99021 may be included in the medium at a concentration of 2 µM to 8 µM, and specifically may be included in the medium at a concentration of 2 µM to 8 µM, 2 µM to 7.5 µM, 2 µM to 7 µM, 2 µM to 6.5 µM, 2 µM to 6 µM, 2 µM to 5.5 µM, 2.5 µM to 8 µM, 2.5 µM to 7.5 µM, 2.5 µM to 7 µM, 2.5 µM to 6.5 µM, 2.5 µM to 6 µM, 2.5 µM to 5.5 µM, 3 µM to 8 µM, 3 µM to 7.5 µM, 3 µM to 7 µM, 3 µM to 6.5 µM, 3 µM to 6 µM, 3 µM to 5.5 µM, 3.5 µM to 8 µM, 3.5 µM to 7.5 µM, 3.5 µM to 7 µM, 3.5 µM to 6.5 µM, 3.5 µM to 6 µM, 3.5 µM to 5.5 µM, 4 µM to 8 µM, 4 µM to 7.5 µM, 4 µM to 7 µM, 4 µM to 6.5 µM, 4 µM to 6 µM, 4 µM to 5.5 µM, 4.5 µM to 8 µM, 4.5 µM to 7.5 µM, 4.5 µM to 7 µM, 4.5 µM to 6.5 µM, 4.5 µM to 6 µM, or 4.5 µM to 5.5 µM.

Additionally, the medium in the primary culturing may not include BMP4 and bFGF, and specifically only the GSK inhibitor may be added to the basic culture medium.

The primary culturing may be culturing of pluripotent stem cells for 24 hours to 72 hours, specifically may be culturing for 24 hours to 72 hours, 24 hours to 66 hours, 24 hours to 60 hours, 24 hours to 54 hours, 30 hours to 72 hours, 30 hours to 66 hours, 30 hours to 60 hours, 30 hours to 54 hours, 36 hours to 72 hours, 36 hours to 66 hours, 36 hours to 60 hours, 36 hours to 54 hours, 42 hours to 72 hours, 42 hours to 66 hours, 42 hours to 60 hours, or 42 hours to 54 hours.

In the method, (B) is secondary culturing of the primary cultured cells, and specifically may be induction or differentiation of the mesoderm (mesodermal cells) differentiated from the pluripotent stem cells into hemangioblasts.

As used herein, the term "hemangioblast" is a multipotent precursor cell that may differentiate into a hematopoietic stem cell or an endothelial cell. In mouse embryos, hematopoiesis begins when blood islands appear in the yolk sac on the 7th day of embryogenesis, and hematopoietic cells and vascular structures are formed from the blood islands. Hemangioblasts are progenitor cells that form blood islands.

The secondary culturing may be culturing of the primary cultured cella or the mesoderm (mesodermal cells) differentiated from the pluripotent stem cells in a medium including one or more selected from the group consisting of bone morphogenetic protein 4 (BMP4), vascular endothelial growth factor (VEGF), and basic fibroblast growth factor (bFGF), and specifically may be culturing in a medium including BMP4, VEGF, and bFGF. The medium may be a hemangioblast differentiation/induction medium.

The BMP4 may be included in the medium at a concentration of 20 ng/ml to 80 ng/ml, and specifically may be included in the medium at a concentration of 20 ng/ml to 80 ng/ml, 20 ng/ml to 75 ng/ml, 20 ng/ml to 70 ng/ml, 20 ng/ml to 65 ng/ml, 20 ng/ml to 60 ng/ml, 20 ng/ml to 55 ng/ml, 25 ng/ml to 80 ng/ml, 25 ng/ml to 75 ng/ml, 25 ng/ml to 70 ng/ml, 25 ng/ml to 65 ng/ml, 25 ng/ml to 60 ng/ml, 25 ng/ml to 55 ng/ml, 30 ng/ml to 80 ng/ml, 30 ng/ml to 75 ng/ml, 30 ng/ml to 70 ng/ml, 30 ng/ml to 65 ng/ml, 30 ng/ml to 60 ng/ml, 30 ng/ml to 55 ng/ml, 35 ng/ml to 80 ng/ml, 35 ng/ml to 75 ng/ml, 35 ng/ml to 70 ng/ml, 35 ng/ml to 65 ng/ml, 35 ng/ml to 60 ng/ml, 35 ng/ml to 55 ng/ml, 40 ng/ml to 80 ng/ml, 40 ng/ml to 75 ng/ml, 40 ng/ml to 70 ng/ml, 40 ng/ml to 65 ng/ml, 40 ng/ml to 60 ng/ml, 40 ng/ml to 55 ng/ml, 45 ng/ml to 80 ng/ml, 45 ng/ml to 75 ng/ml, 45 ng/ml to 70 ng/ml, 45 ng/ml to 65 ng/ml, 45 ng/ml to 60 ng/ml, or 45 ng/ml to 55 ng/ml.

The bFGF may be included in the medium at a concentration of 80 ng/ml to 120 ng/ml, and specifically may be included in the medium at a concentration of 80 ng/ml to 120 ng/ml, 80 ng/ml to 115 ng/ml, 80 ng/ml to 110 ng/ml, 80 ng/ml to 105 ng/ml, 85 ng/ml to 120 ng/ml, 85 ng/ml to 115 ng/ml, 85 ng/ml to 110 ng/ml, 85 ng/ml to 105 ng/ml, 90 ng/ml to 120 ng/ml, 90 ng/ml to 115 ng/ml, 90 ng/ml to 110 ng/ml, 90 ng/ml to 105 ng/ml, 95 ng/ml to 120 ng/ml, 95 ng/ml to 115 ng/ml, 95 ng/ml to 110 ng/ml, or 95 ng/ml to 105 ng/ml.

The VEGF may be included in the medium at a concentration of 20 ng/ml to 80 ng/ml, and specifically may be included at a concentration of 20 ng/ml to 80 ng/ml, 20 ng/ml to 75 ng/ml, 20 ng/ml to 70 ng/ml, 20 ng/ml to 65 ng/ml, 20 ng/ml to 60 ng/ml, 20 ng/ml to 55 ng/ml, 25 ng/ml to 80 ng/ml, 25 ng/ml to 75 ng/ml, 25 ng/ml to 70 ng/ml, 25 ng/ml to 65 ng/ml, 25 ng/ml to 60 ng/ml, 25 ng/ml to 55 ng/ml, 30 ng/ml to 80 ng/ml, 30 ng/ml to 75 ng/ml, 30 ng/ml to 70 ng/ml, 30 ng/ml to 65 ng/ml, 30 ng/ml to 60 ng/ml, 30 ng/ml to 55 ng/ml, 35 ng/ml to 80 ng/ml, 35 ng/ml to 75 ng/ml, 35 ng/ml to 70 ng/ml, 35 ng/ml to 65 ng/ml, 35 ng/ml to 60 ng/ml, 35 ng/ml to 55 ng/ml, 40 ng/ml to 80 ng/ml, 40 ng/ml to 75 ng/ml, 40 ng/ml to 70 ng/ml, 40 ng/ml to 65 ng/ml, 40 ng/ml to 60 ng/ml, 40 ng/ml to 55 ng/ml, 45 ng/ml to 80 ng/ml, 45 ng/ml to 75 ng/ml, 45 ng/ml to 70 ng/ml, 45 ng/ml to 65 ng/ml, 45 ng/ml to 60 ng/ml, or 45 ng/ml to 55 ng/ml.

The secondary culturing may be culturing for 24 hours to 72 hours, and specifically may be culturing for 24 hours to 72 hours, 24 hours to 66 hours, 24 hours to 60 hours, 24 hours to 54 hours, 30 hours to 72 hours, 30 hours to 66 hours, 30 hours to 60 hours, 30 hours to 54 hours, 36 hours to 72 hours, 36 hours to 66 hours, 36 hours to 60 hours, 36 hours to 54 hours, 42 hours to 72 hours, 42 hours to 66 hours, 42 hours to 60 hours, or 42 hours to 54 hours.

In the method, (C) is thirdly culturing the secondary cultured cells, and specifically may be induction of an endothelial-to-hematopoietic Transition (EHT) of the hemangioblasts differentiated from the mesoderm (mesodermal cells) to obtain EHT-induced hemangioblasts.

As used herein, the term "endothelial-to-hematopoietic transition (EHT)" refers to a process of transition from endothelial characteristics to hematopoietic characteristics, and specifically refers to the induction of an increase in hematopoietic proficiency from cells exhibiting endothelial characteristics.

The thirdly culturing may be culturing of the secondary cultured cells or the hemangioblasts differentiated from the mesoderm in a medium including a TGF-beta inhibitor, and the medium may be an EHT induction medium.

As used herein, the term "TGF-beta inhibitor" refers to a molecule that inhibits the TGF-beta pathway by inhibiting the interaction between TGF-beta and a TGF-beta receptor (TGF-betaR). The TGF-beta inhibitor may include one or more selected from the group consisting of SB-431542 (SB4), galunisertib, LY2109761, SB525334, SP505124, GW788388, LY364947, RepSox, SD-208, vactosertib, LY3200882, PF-06952229, and pharmaceutically acceptable salt thereofs, specifically may be SB-431542 (SB4) or a pharmaceutically acceptable salt thereof.

As used herein, the term "SB-431542 (SB4)" is known as a TGF-beta inhibitor and is represented by the chemical formula C₂₂H₁₆N₄O₃, and may be expressed by the structural formula of Formula 2 below.

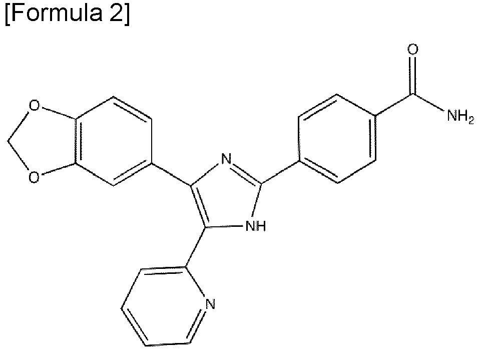

The SB-431542 may be included in the medium at a concentration of 7 µM to 13 µM, and specifically may be included in the medium at a concentration of 7 µM to 13 µM, 7 µM to 12.5 µM, 7 µM to 12 µM, 7 µM to 11.5 µM, 7 µM to 11 µM, 7 µM to 10.5 µM, 7.5 µM to 13 µM, 7.5 µM to 12.5 µM, 7.5 µM to 12 µM, 7.5 µM to 11.5 µM, 7.5 µM to 11 µM, 7.5 µM to 10.5 µM, 8 µM to 13 µM, 8 µM to 12.5 µM, 8 µM to 12 µM, 8 µM to 11.5 µM 8 µM to 11 µM, 8 µM to 10.5 µM, 8.5 µM to 13 µM, 8.5 µM to 12.5 µM, 8.5 µM to 12 µM, 8.5 µM to 11.5 µM, 8.5 µM to 11 µM, 8.5 µM to 10.5 µM, 9 µM to 13 µM, 9 µM to 12.5 µM, 9 µM to 12 µM, 9 µM to 11.5 µM, 9 µM to 11 µM, 9 µM to 10.5 µM, 9.5 µM to 13 µM, 9.5 µM to 12.5 µM, 9.5 µM to 12 µM, 9.5 µM to 11.5 µM, 9.5 µM to 11 µM, or 9.5 µM to 10.5 µM.

The medium of the thirdly culturing may further include retinoic acid (RA) or a pharmaceutically acceptable salt thereof.

The retinoic acid may be included in the medium at a concentration of 0.5 µM to 1.5 µM, and specifically may be included in the medium at a concentration of 0.5 µM to 1.5 µM, 0.5 µM to 1.3 µM, 0.5 µM to 1.1 µM, 0.7 µM to 1.5 µM, 0.7 µM to 1.3 µM, 0.7 µM to 1.1 µM, 0.9 µM to 1.5 µM, 0.9 µM to 1.3 µM, or 0.9 µM to 1.1 µM.

The medium of the thirdly culturing may further include one or more selected from the group consisting of VEGF and bFGF, and specifically may further include VEGF and bFGF.

The VEGF and bFGF may be included in the medium at a concentration of 20 ng/ml to 80 ng/ml, respectively, and specifically may be included in the medium at a concentration of 20 ng/ml to 80 ng/ml, 20 ng/ml to 75 ng/ml, 20 ng/ml to 70 ng/ml, 20 ng/ml to 65 ng/ml, 20 ng/ml to 60 ng/ml, 20 ng/ml to 55 ng/ml, 25 ng/ml to 80 ng/ml, 25 ng/ml to 75 ng/ml, 25 ng/ml to 70 ng/ml, 25 ng/ml to 65 ng/ml, 25 ng/ml to 60 ng/ml /ml, 25 ng/ml to 55 ng/ml, 30 ng/ml to 80 ng/ml, 30 ng/ml to 75 ng/ml, 30 ng/ml to 70 ng/ml, 30 ng/ml to 65 ng/ml, 30 ng/ml to 60 ng/ml, 30 ng/ml to 55 ng/ml, 35 ng/ml to 80 ng/ml, 35 ng/ml to 75 ng/ml, 35 ng/ml to 70 ng/ml, 35 ng/ml to 65 ng/ml, 35 ng/ml to 60 ng/ml, 35 ng/ml to 55 ng/ml, 40 ng/ml to 80 ng/ml , 40 ng/ml to 75 ng/ml, 40 ng/ml to 70 ng/ml, 40 ng/ml to 65 ng/ml, 40 ng/ml to 60 ng/ml, 40 ng/ml to 55 ng/ml, 45 ng/ml to 80 ng/ml, 45 ng/ml to 75 ng/ml, 45 ng/ml to 70 ng/ml, 45 ng/ml to 65 ng/ml, 45 ng/ml to 60 ng/ml, or 45 ng/ml to 55 ng/ml.

The thirdly culturing may be culturing for 12 hours to 44 hours, specifically may be of culturing for 12 hours to 44 hours, 12 hours to 40 hours, 12 hours to 36 hours, 12 hours to 32 hours, 12 hours to 28 hours, 16 hours to 44 hours, 16 hours to 40 hours, 16 hours to 36 hours, 16 hours to 32 hours, 16 hours to 28 hours, 20 hours to 44 hours, 20 hours to 40 hours, 20 hours to 36 hours, 20 hours to 32 hours, or 20 hours to 28 hours.

In the method, (D) is fourthly culturing of the third cultured cells, and may be differentiation/induction of hemangioblasts, specifically EHT-induced hemangioblasts into hematopoietic stem cells. The differentiated hematopoietic stem cells may be CD34⁺ hematopoietic stem cells.

The fourthly culturing may be culturing of the thirdly cultured cells or the EHT-induced hemangioblasts in a medium including polyvinyl alcohol (PVA) or a pharmaceutically acceptable salt thereof. The medium may be a hematopoietic stem cell differentiation/induction medium.

The PVA may be included in the medium at a concentration of 0.01 % (w/v) to 0.5 % (w/v), and specifically may be included in the medium at a concentration of 0.01 (w/v) % to 0.5 % (w/v), 0.01 % (w/v) to 0.4 % (w/v), 0.01 % (w/v) to 0.3% (w/v), 0.01 % (w/v) to 0.2 % (w/v), 0.01 % (w/v) to 0.15 % (w/v), 0.03 % (w/v) to 0.5 % (w/v), 0.03 % (w/v) to 0.4 % (w/v), 0.03 % (w/v) to 0.3 % (w/v), 0.03 % (w/v) to 0.2 % (w/v), 0.03 % (w/v) to 0.15 % (w/v), 0.05 % (w/v) to 0.5 % (w/v), 0.05 % (w/v) to 0.4 % (w/v), 0.05 % (w/v) to 0.3 % (w/v), 0.05 % (w/v) to 0.2 % (w/v), 0.05 % (w/v) to 0.15 % (w/v), 0.07 % (w/v) to 0.5 % (w/v), 0.07 % (w/v) to 0.4 % (w/v), 0.07 % (w/v) to 0.3 % (w/v), 0.07 % (w/v) to 0.2% (w/v), 0.07 % (w/v) to 0.15 % (w/v), 0.09 % (w/v) to 0.5 % (w/v), 0.09 % (w/v) to 0.4 % (w/v), 0.09 % (w/v) to 0.3 % (w/v), 0.09 % (w/v) to 0.2 % (w/v), or 0.09 % (w/v) to 0.15 % (W/V).

The medium of the fourthly culturing may further include one or more selected from the group consisting of stem cell factor (SCF) and bFGF, and specifically may further include SCF and bFGF.

The SCF may be included in the medium at a concentration of 20 ng/ml to 80 ng/ml, and specifically may be included in the medium at a concentration of 20 ng/ml to 80 ng/ml, 20 ng/ml to 75 ng/ml, 20 ng/ml to 70 ng/ml, 20 ng/ml to 65 ng/ml, 20 ng/ml to 60 ng/ml, 20 ng/ml to 55 ng/ml, 25 ng/ml to 80 ng/ml, 25 ng/ml to 75 ng/ml, 25 ng/ml to 70 ng/ml, 25 ng/ml to 65 ng/ml, 25 ng/ml to 60 ng/ml, 25 ng/ml to 55 ng/ml, 30 ng/ml to 80 ng/ml, 30 ng/ml to 75 ng/ml, 30 ng/ml to 70 ng/ml, 30 ng/ml to 65 ng/ml, 30 ng/ml to 60 ng/ml, 30 ng/ml to 55 ng/ml, 35 ng/ml to 80 ng/ml, 35 ng/ml to 75 ng/ml, 35 ng/ml to 70 ng/ml, 35 ng/ml to 65 ng/ml, 35 ng/ml to 60 ng/ml, 35 ng/ml to 55 ng/ml, 40 ng/ml to 80 ng/ml, 40 ng/ml to 75 ng/ml, 40 ng/ml to 70 ng/ml, 40 ng/ml to 65 ng/ml, 40 ng/ml to 60 ng/ml, 40 ng/ml to 55 ng/ml, 45 ng/ml to 80 ng/ml, 45 ng/ml to 75 ng/ml, 45 ng/ml to 70 ng/ml, 45 ng/ml to 65 ng/ml, 45 ng/ml to 60 ng/ml, or 45 ng/ml to 55 ng/ml.

The bFGF may be included in the medium at a concentration of 7 ng/ml to 13 ng/ml, and specifically may be included in the medium at a concentration of 7 ng/ml to 13 ng/ml, 7 ng/ml to 12.5 ng/ml, 7 ng/ml to 12 ng/ml, 7 ng/ml to 11.5 ng/ml, 7 ng/ml to 11 ng/ml, 7 ng/ml to 10.5 ng/ml, 7.5 ng/ml to 13 ng/ml, 7.5 ng/ml to 12.5 ng/ml, 7.5 ng/ml to 12 ng/ml, 7.5 ng/ml to 11.5 ng/ml, 7.5 ng/ml to 11 ng/ml, 7.5 ng/ml to 10.5 ng/ml, 8 ng/ml to 13 ng/ml, 8 ng/ml to 12.5 ng/ml, 8 ng/ml to 12 ng/ml, 8 ng/ml to 11.5 ng/ml, 8 ng/ml to 11 ng/ml, 8 ng/ml to 10.5 ng/ml, 8.5 ng/ml to 13 ng/ml, 8.5 ng/ml to 12.5 ng/ml, 8.5 ng/ml to 12 ng/ml, 8.5 ng/ml to 11.5 ng/ml, 8.5 ng/ml to 11 ng/ml, 8.5 ng/ml to 10.5 ng/ml, 9 ng/ml to 13 ng/ml, 9 ng/ml to 12.5 ng/ml, 9 ng/ml to 12 ng/ml, 9 ng/ml to 11.5 ng/ml, 9 ng/ml to 11 ng/ml, 9 ng/ml to 10.5 ng/ml, 9.5 ng/ml to 13 ng/ml, 9.5 ng/ml to 12.5 ng/ml, 9.5 ng/ml to 12 ng/ml, 9.5 ng/ml to 11.5 ng/ml, 9.5 ng/ml to 11 ng/ml, or 9.5 ng/ml to 10.5 ng/ml.

The medium of the fourthly culturing may not include one or more selected from the group consisting of VEGF, IL-3, and IL-6, and specifically may not include VEGF, IL-3, and IL-6.

The fourthly culturing may be culturing for 168 hours to 360 hours, and specifically may be culturing for 168 hours to 360 hours, 168 hours to 348 hours, 168 hours to 336 hours, 168 hours to 324 hours, 168 hours to 312 hours, 168 hours to 300 hours, 168 hours to 288 hours, 192 hours to 360 hours, 192 hours to 348 hours, 192 hours to 336 hours, 192 hours to 324 hours, 192 hours to 312 hours, 192 hours to 300 hours, 192 hours to 288 hours, 216 hours to 360 hours, 216 hours to 348 hours, 216 hours to 336 hours, 216 hours to 324 hours, 216 hours to 312 hours, 216 hours to 300 hours, 216 hours to 288 hours, 240 hours to 360 hours, 240 hours to 348 hours, 240 hours to 336 hours, 240 hours to 324 hours, 240 hours to 312 hours, 240 hours to 300 hours, or 240 hours to 288 hours.

The method of preparing hematopoietic stem cells may further include culturing to maintain hematopoietic stem cells differentiated from pluripotent stem cells.

The culturing to maintain the hematopoietic stem cells is for maintaining the biological/physiological characteristics of the differentiated hematopoietic stem cells, and specifically may be for maintaining CD34+ potential by inhibiting aging and differentiation of the differentiated hematopoietic stem cells.

The culturing for maintaining may use the medium of the fourthly culturing, and may be culturing for 1 to 20 days.

The method may further include recovering the fourthly cultured cells, specifically recovering the hematopoietic stem cells differentiated from the pluripotent stem cells.

The basic medium used in the method of preparing hematopoietic stem cells may be a conventional medium known in the art to be suitable for stem cell culture and differentiation, and examples include, but are not limited to, Dulbecco's Modified Eagle's Medium (DMEM), minimal essential medium (MEM), Basal Medium Eagle (BME), RPM11640, F-10, F-12, α-minimal essential medium (α-MEM), Glasgow's minimal essential medium (GMEM), Iscove's Modified Dulbecco's Medium, Stempro 34-SFM and Stempro 34.

The basic medium may be supplemented with additives. In general, the basic medium may include neutral buffering agents (including phosphate and/or high concentration bicarbonate) and protein nutrients (including serum, selected from Fetal Bovine Serum (FBS), serum substitute, albumin, or essential and non-essential amino acids including glutamine) in isotonic solution. Furthermore, the basic medium may include lipids (high-density lipoprotein (HDL) or low-density lipoprotein (LDL) extracts from fatty acids, cholesterol, or serum) and other components found in most preservative media of this type (for example, insulin or transferrin, nucleosides or nucleotides, pyruvate, glycogen in any ionized form or salt, including glucose, selenium, glucocorticoids such as hydrocortisone, and/or reducing agents such as β-mercaptoethanol). Additionally, the medium may include antibiotics such as penicillin, streptomycin, gentamicin, or a mixture of two or more thereof.

The method of preparing hematopoietic stem cells involves differentiating pluripotent stem cells into hematopoietic stem cells, and may include 1) culturing the pluripotent stem cells in a medium including 4 µM to 6 µM of CHIR99021 for about 2 days to differentiate/induce into mesoderm (mesodermal cells/ mesodermal stem cells), 2) culturing the differentiated mesoderm in a medium including 40 ng/ml to 60 ng/ml of BMP4, 90 ng/ml to 100 ng/ml of bFGF and 40 ng/ml to 60 ng/ml of VEGF for about 2 days to differentiate/induce into hemangioblasts, 3) culturing the hemangioblasts for about 1 day in a medium including 40 ng/ml to 60 ng/ml of bFGF, 40 ng/ml to 60 ng/ml of VEGF, 9 µM to 11 µM of SB-431542, and 0.8 µM to 1.2 µM of retinoic acid to induce EHT, and 4) culturing the EHT-induced hemangioblasts in a medium including 0.05 % to 0.15 % of PVA, 40 ng/ml to 60 ng/ml of SCF, and 9 ng/ml to 11 ng/ml of bFGF for about 11 days to differentiate/induce it into hematopoietic stem cells. Additionally, a Stempro 34-SFM (+ stempro supplement) medium including 200 ug/ml of human transferrin, 2 mM of L-glutamine, 0.5 mM of L-Ascorbic acid, 0.45 mM of MTG (1-thioglycerol) and 1% of penicillin/streptomycin may be used as a basic culture medium.

Another aspect provides a method of constructing a humanized animal model, including transplanting or injecting hematopoietic stem cells prepared by the method of preparing hematopoietic stem cells into an individual other than a human. The content overlapping with the above-described content also applies to the method.

The humanized animal model may be a mammal such as a primate, mouse, hamster, guinea pig, rat, dog, cat, horse, and cow, and may specifically be a mouse.

The transplanting or injecting of the hematopoietic stem cells may be injecting of the pluripotent stem cell-derived hematopoietic stem cells prepared by the method into a tail vein of a mouse, and specifically, may be injecting 1 × 10⁵ to 2 × 10⁵ cells.

The individual other than a human may be an individual with induced immune deficiency due to a complete lack of function of T cells, B cells, NK cells and the like, specifically may be an irradiated NOD scid gamma (NSG) mouse.

Another aspect provides a humanized animal model constructed by the method of constructing a humanized animal model. The content overlapping with the above-described content also applies to the animal model.

The humanized animal model may be a mammal such as a primate, mouse, hamster, guinea pig, rat, dog, cat, horse, and cow, and specifically may be a mouse.

### Advantageous Effects

According to an aspect, the method identified an optimal differentiation condition according to the combination of low-molecular-weight compounds and protein growth factors without gene insertion, and may differentiate hematopoietic stem cells from pluripotent stem cells with high efficiency.

### Description of Drawings

FIG. 1 is a diagram schematically showing a novel pluripotent stem cell-derived hematopoietic stem cell differentiation protocol of the present disclosure.
FIG. 2 is a diagram specifically describing a pluripotent stem cell-derived hematopoietic stem cell differentiation protocol of the present disclosure.
FIG. 3 is a diagram showing the results of a comparative experiment to establish the primary conditions of inducing hematopoietic stem cells.
FIG. 4 is a diagram showing the results of a comparative experiment to establish the conditions mesoderm induction.
FIG. 5 is a diagram showing the results of a comparative experiment to establish the conditions of inducing EHT.
FIG. 6 is a diagram showing the results of a comparative experiment to establish the secondary conditions of inducing hematopoietic stem cells.
FIG. 7 is a diagram showing a process of constructing a humanized mouse model by using hematopoietic stem cells prepared by a method of the present disclosure.

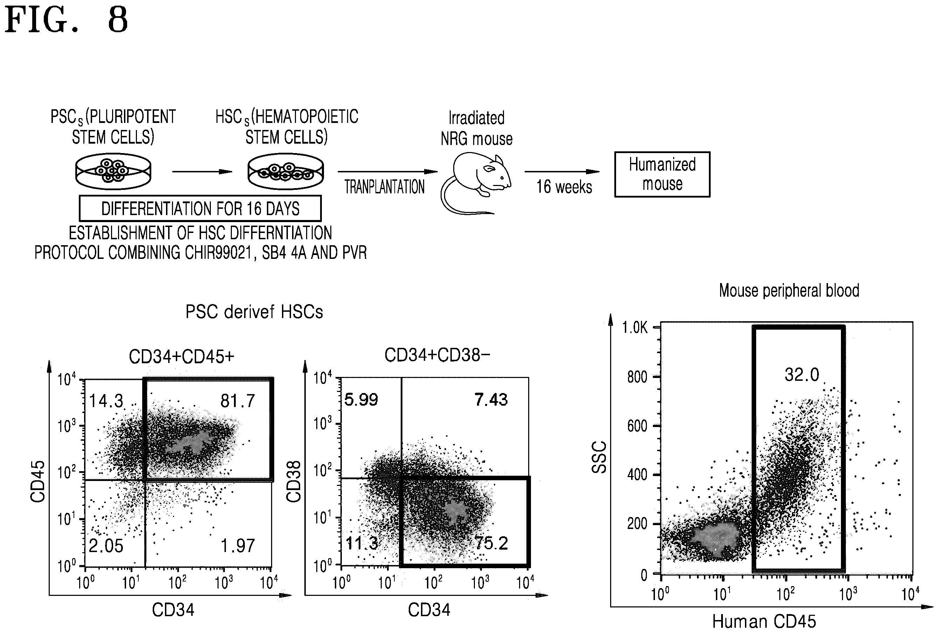

FIG. 8 is a diagram schematically showing a method of preparing hematopoietic stem cells and a method of constructing a humanized mouse model by using the same, of the present disclosure.

### Mode for Invention

Hereinafter, it will be described in more detail through examples. However, these examples are for illustrative purposes only and the scope of the present disclosure is not limited to these examples.

### Example 1: Novel human pluripotent stem cell-derived hematopoietic stem cell differentiation protocol

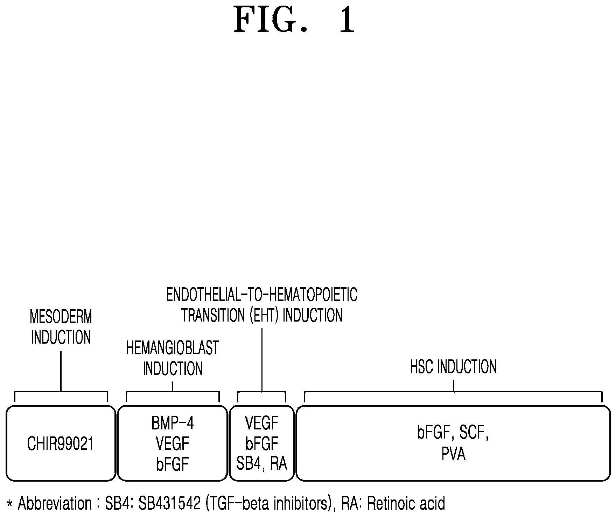

The present disclosure relates to a method of differentiating human pluripotent stem cells to prepare human hematopoietic stem cells, and a method of constructing a humanized mice by using hematopoietic stem cells prepared by the method. The method may be composed of 1) mesoderm induction/differentiation, 2) hemangioblast induction/differentiation, 3) induction of endothelial-to-hematopoietic transition (EHT), and 4) hematopoietic stem cell (HSC) induction/differentiation, and in each step, treatment with essential low-molecular-weight compounds and/or growth factors (FIG. 1) may be carried out.

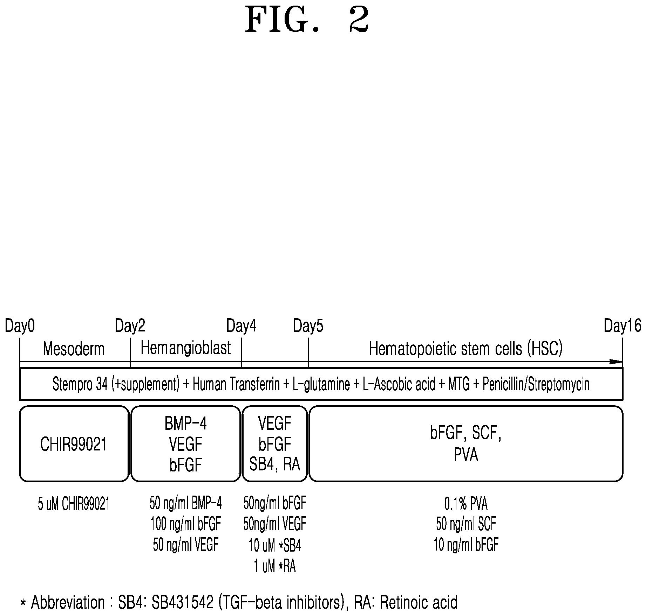

Specific experiments on the method of preparing human hematopoietic stem cells were performed as follows. First, isolated human pluripotent stem cells (hPSCs) were maintained in stemMACS-iPS brew medium for 2 days. The differentiation base culture medium consisted of the following composition: Stempro 34-SFM (+ stempro sup) + 200 ug/ml of human Transferrin + 2 mM of L-glutamine + 0.5 mM of L-ascorbic acid + 0.45 mM of MTG (1-thioglycerol) + 1% of penicillin/streptomycin. To induce mesodermfrom the isolated hPSCs, treatment was performed with CHIR99021 (5uM) alone, a representative example of a GSK inhibitor, for about 2 days. Next, treatment was performed with 50 ng/ml of BMP4, 50 ng/ml of VEGF, and 100 ng/ml of bFGF for 2 days to induce hemangioblasts. Next, 50 ng/ml of VEGF, 50 ng/ml of bFGF, and SB-431542 (10 µM) as representative examples of TGF-beta inhibitors and 1 µM of retinoic acid (RA) were added and cultured for about 1 day, and 0.1% (w/v) of PVA, 50 ng/ml of stem cell factor (SCF), and 10 ng/ml of bFGF were added and cultured for about 11 days (FIG. 2).

In most of the previously published technologies related to hematopoietic stem cell differentiation, differentiation was carried out by forming a colony from hPSCs to Embryonic bodies (EBs), breaking up the colony into single cells, and extracting CD34+ HSCs, and gene insertion was attempted to increase differentiation efficiency. However, the HSC differentiation method of the present disclosure has the excellent effect of being able to differentiate HSCs with high efficiency and easily obtain HSCs only with an optimal combination of low-molecular-weight compounds and protein growth factors, without the complicated processes mentioned above and without gene insertion.

Hereinafter, in order to develop a novel hematopoietic stem cell differentiation method of the present disclosure, experiments were performed to establish optimal conditions while comparing with previously published differentiation protocols.

### Example 2: Establishment of primary conditions of inducing hematopoietic stem cells

To establish the primary conditions of inducing hematopoietic stem cells, the following experiment was performed.

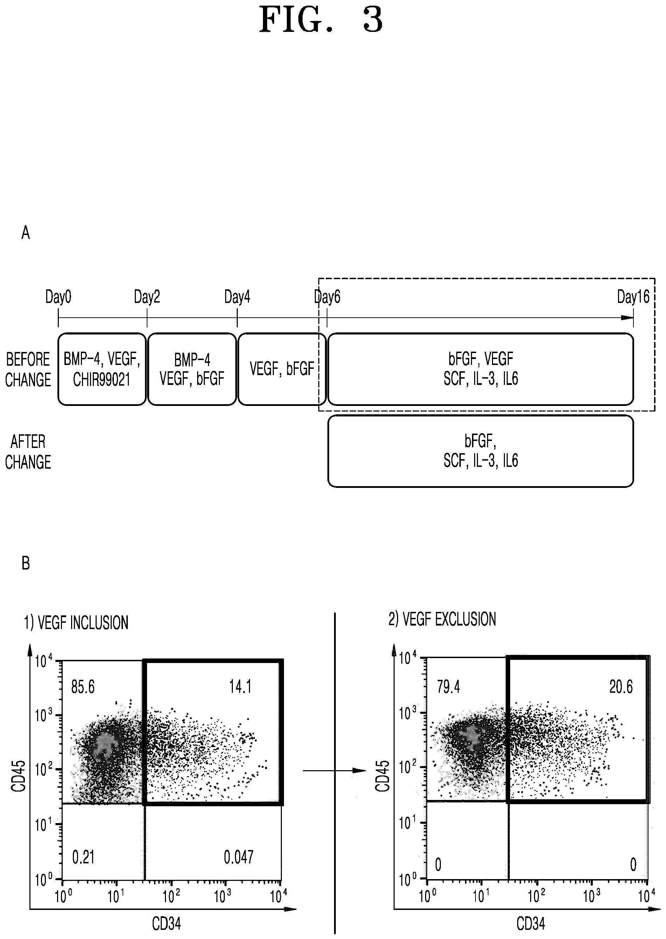

Specifically, it is widely known that vascular endothelial growth factor (VEGF) may be used to induce mesoderm and induce and maintain hemangioblasts (precursors of HSCs), and has been mainly used during the period of inducing hematopoietic stem cells. Therefore, to determine what effect it will have on the final hematopoietic stem cell generation efficiency when VEGF is excluded from the induction of hematopoietic stem cells, the results of hematopoietic stem cell differentiation according to the presence or absence of VEGF (10 ng/ml) were confirmed under the same conditions (50 ng/ml of SCF, 10 ng/ml of bFGF, 20 ng/ml of IL-3 and 10 ng/ml of IL-6) (FIG. 3A).

To confirm the differentiation results, Fluorescence Activated Cell Sorting (FACS) was performed to confirm the proportion of CD34⁺CD45⁺ cells. Specifically, to identify cells expressing both CD34 and CD45, anti-CD34 and anti-CD45 antibodies linked with the fluorescent substances PE and PerCP/Cy5.5, respectively, were diluted in staining buffer (phosphate buffered saline (PBS) containing 1% FBS) at a ratio of 1:25. Then, the cells were stained with a cocktail containing the antibodies for 35 minutes at 4 °C. After washing away the remaining antibodies with staining buffer, 200 µl of staining buffer was added again and analyzed using a flow cytometer.

As a result, it was confirmed that the ratio of CD34⁺CD45⁺ cells in the case of excluding VEGF from the induction of hematopoietic stem cells was significantly superior compared to the case in which VEGF was included (FIG. 3B). Based on the above results, it may be seen that, unlike what was previously known, excluding VEGF from the induction of hematopoietic stem cells has a remarkably excellent hematopoietic stem cell differentiation effect.

### Example 3: Establishment of conditions of inducing mesoderm

To establish the conditions of inducing mesoderm (mesodermalcells/mesodermalstem cells), the following experiment was performed.

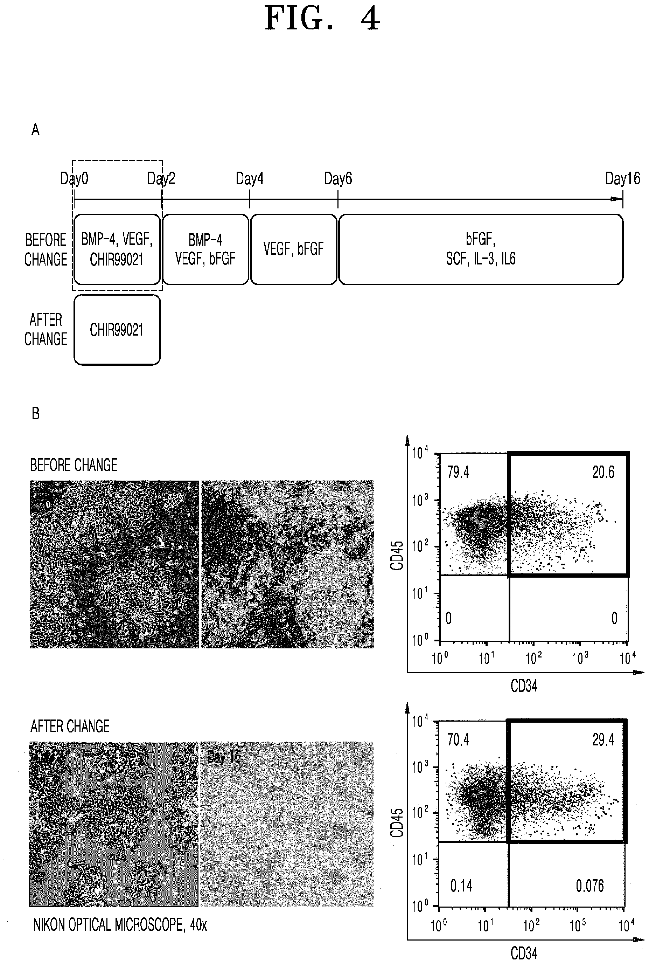

Specifically, in the existing method, treatment was performed with bone morphogenetic protein 4 (BMP4), VEGF, and CHIR99021 to induce mesoderm. However, it was determined that the above conditions were not compatible with the sequence of the process for differentiating stem cells into hematopoietic stem cells. Therefore, the hematopoietic stem cell differentiation efficiency was confirmed when treating only with CHIR99021 as a representative example of a GSK inhibitor in inducing mesoderm, excluding BMP4 (5 ng/ml) and VEGF (50 ng/ml), which were previously used for treatment (FIG. 4A).

As a result, it was confirmed that the ratio of CD34⁺CD45⁺ cells when treated with CHIR99021 alone in inducing mesodermwas significantly superior compared to when treated with BMP4, VEGF, and CHIR99021 (FIG. 4B). Based on the above results, it may be seen that not treating with BMP4 and VEGF in inducing mesodermhas a remarkably excellent hematopoietic stem cell differentiation effect.

### Example 4: Establishment of conditions of inducing EHT

To establish the conditions of inducing endothelial-to-hematopoietic transition (EHT), the following experiment was performed.

Specifically, in order to induce CD34+ HSCs, hemangioblasts must be induced after inducing the mesoderm. Since hemangioblasts may be differentiated into CD34+ HSCs by inducing endothelial-to-hematopoietic transition (EHT), it is important to establish appropriate culture conditions to maximize the efficiency of EHT.

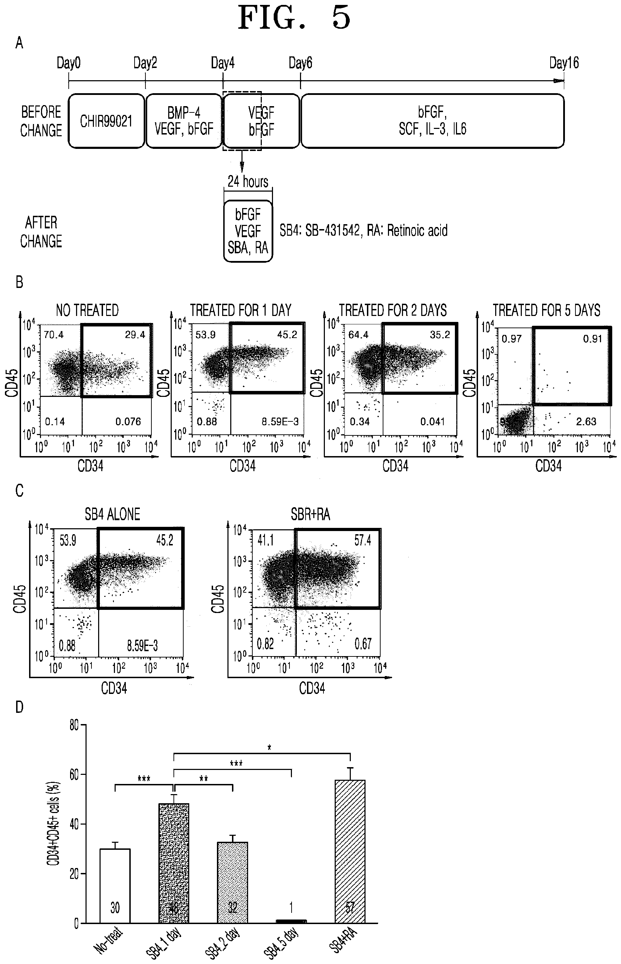

For this purpose, the efficiency of hematopoietic stem cell differentiation when treated with SB-431542 (SB4), a representative example of a TGF-beta inhibitor, in addition to VEGF and basic fibroblast growth factor (bFGF) for various periods of time, was determined (FIG. 5A). As a result, it was confirmed that when treated with SB4 for less than 24 hours, the proportion of CD34+ CD45+ cells was significantly superior, and when treated with SB4 for a long period of time, the hematopoietic stem cell differentiation efficiency was significantly reduced (FIG. 5B). In addition, when treated simultaneously with SB4 and retinoic acid (RA), it was confirmed that the hematopoietic stem cell differentiation efficiency was significantly increased compared to when treated with SB4 alone (FIGS. 5C and 5D).

Based on the above results, it may be seen that inducing EHT after inducing hemangioblasts has a significantly better hematopoietic stem cell differentiation effect when treated with SB4 for about 24 hours, and the combination of SB4 and retinoic acid shows a better hematopoietic stem cell differentiation effect.

### Example 5: Establishment of secondary conditions of inducing hematopoietic stem cells

To establish the secondary conditions of inducing hematopoietic stem cells, the following experiment was performed.

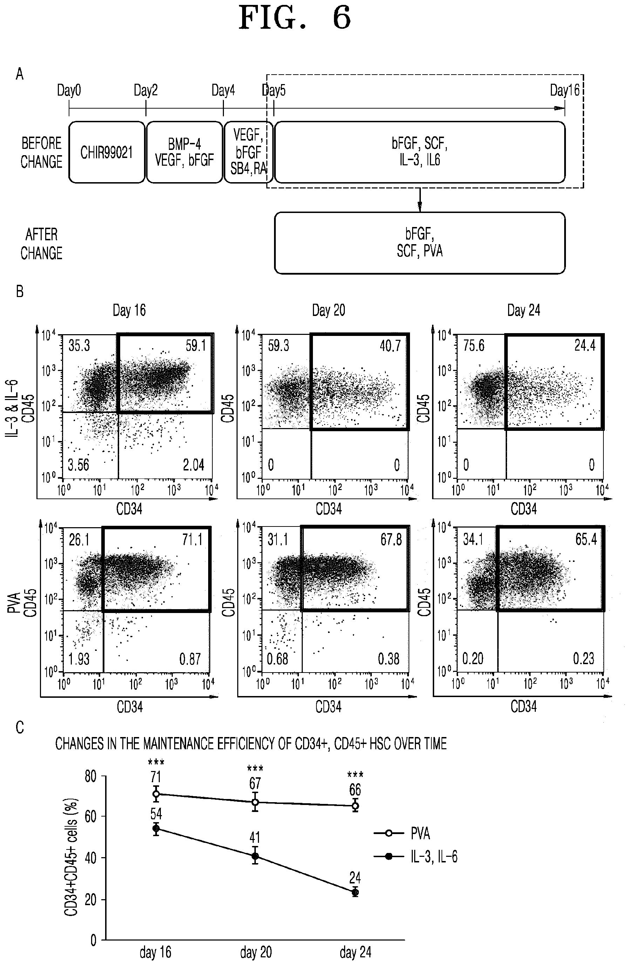

Specifically, IL-3 and IL-6 are mainly used in the culture process to maintain the CD34+ potential of hematopoietic stem cells differentiated from human pluripotent stem cells, but this may lead to aging and differentiation of differentiated hematopoietic stem cells, which may slightly reduce the efficiency of maintaining CD34⁺ potential. Therefore, the efficiency of maintaining hematopoietic stem cell differentiation was determined when treating with polyvinyl alcohol (PVA) instead of IL-3 and IL-6, which were previously used for treatment, in inducing and maintaining hematopoietic stem cells (FIG. 6A).

As a result, it was confirmed that when treated with PVA while excluding IL-3 and IL-6 from induction of hematopoietic stem cells, not only did the hematopoietic stem cell differentiation efficiency increase, but the maintenance efficiency of CD34+ hematopoietic stem cells after differentiation was also significantly superior (FIGS. 6B and 6C). Based on the above results, unlike what was previously known, it may be seen that treating with PVA while excluding IL-3 and IL-6 in inducing hematopoietic stem cells, has a significantly superior hematopoietic stem cell differentiation and maintenance effect.

### Example 6: Construction of humanized mice

Based on the experimental results of Examples 2 to 5, the novel hematopoietic stem cell differentiation protocol of Example 1 was established, and humanized mice were constructed using hematopoietic stem cells prepared by the method.

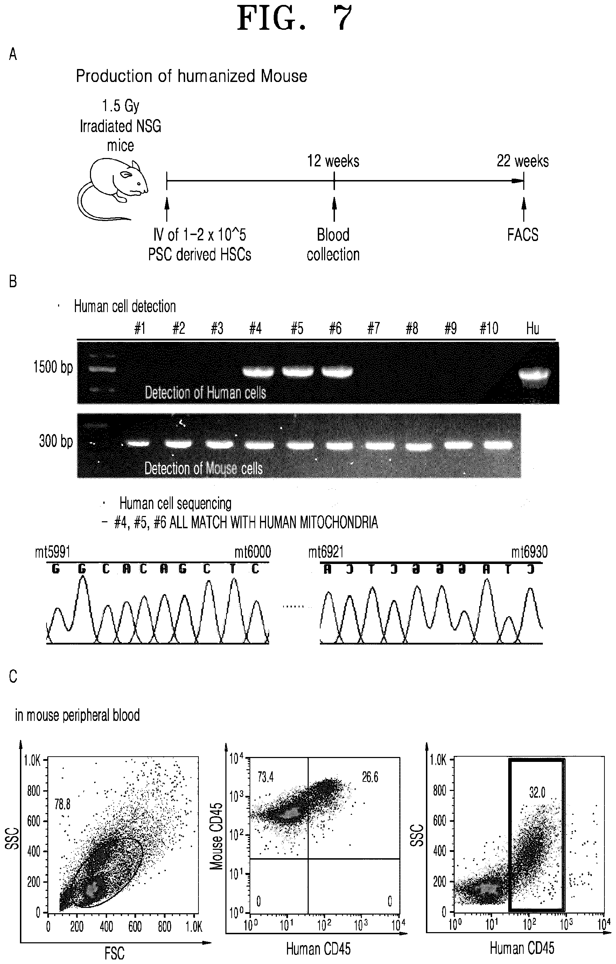

Specifically, 1 × 10⁵ - 2 × 10⁵ of human pluripotent stem cell-derived hematopoietic stem cells prepared according to the method of Example 1 were injected into a tail vein of irradiated immunodeficient NSG mice. The presence of human mitochondrial genes was confirmed in the peripheral blood of mice 12 weeks after injection, and the presence of human CD45-positive cells was confirmed in the peripheral blood of mice 22 weeks after injection (FIG. 7A).

PCR reactions were performed to detect human mitochondrial DNA in the peripheral blood of the mice, specifically, 2x PCRBIO HS Taq Mix red was used as polymerase, and the annealing temperature was set to 58 °C and performed for 35 cycles. Afterwards, the PCR reaction product was loaded onto a 1% agarose gel containing EtBr at 100 v for 20 minutes to confirm the PCR band. The sequence of the primers used to detect human mitochondria is as follows.
Forward: 5'- CAACACTAAAGGACGAACCTGA-3' (SEQ ID NO: 1)
Reverse: 5'- TCGTAAGGGGTGGATTTTTC-3' (SEQ ID NO: 2)

As a result, human mitochondrial genes were detected in the peripheral blood of mice 12 weeks after injection (FIG. 7B), and 22 weeks after injection, it was confirmed that more than 25% of human CD45-positive cells were present in the peripheral blood of mice (FIG. 7C).

Based on the above results, it may be seen that the hematopoietic stem cells prepared by the hematopoietic stem cell differentiation protocol method of Example 1 may also be usefully used to construct a humanized animal model.

The description of the present invention described above is for illustrative purposes, and those skilled in the art will understand that the present disclosure may be easily modified into other specific forms without changing the technical concept or essential features of the present disclosure. Therefore, the embodiments described above should be understood in all respects as illustrative and not restrictive.

## Claims

1. A method of preparing hematopoietic stem cells, comprising:
(A) primary culturing pluripotent stem cells (PSCs) in a medium containing a glycogen synthase kinase (GSK) inhibitor to obtain mesodermal cells;
(B) secondary culturing the mesodermal cells to induce or differentiate the mesodermal cells into hemangioblasts;
(C) thirdly culturing the hemangioblasts to obtain Endothelial-to-Hematopoietic Transition (EHT)-induced hemangioblasts; and
(D) fourthly culturing the EHT-induced hemangioblasts to induce or differentiate the EHT-induced hemangioblasts into the hematopoietic stem cells.

2. The method of claim 1, wherein the glycogen synthase kinase (GSK) inhibitor is CHIR99021.

3. The method of claim 1, wherein the secondary culturing is culturing in a medium including one or more selected from the group consisting of bone morphogenetic protein 4 (BMP4), vascular endothelial growth factor (VEGF), and basic fibroblast growth factor (bFGF).

4. The method of claim 1, wherein the thirdly culturing is culturing in a medium including a TGF-beta inhibitor.

5. The method of claim 4, wherein the TGF-beta inhibitor is SB-431542.

6. The method of claim 4, wherein the thirdly culturing further includes retinoic acid (RA) or a pharmaceutically acceptable salt thereof.

7. The method of claim 4, wherein the thirdly culturing further includes one or more selected from the group consisting of VEGF and bFGF.

8. The method of claim 1, wherein the fourthly culturing is culturing in a medium including polyvinyl alcohol (PVA) or a pharmaceutically acceptable salt thereof.

9. The method of claim 8, wherein the fourthly culturing is culturing in a medium further including one or more selected from the group consisting of SCF and bFGF.

10. A method of constructing a humanized animal model, comprising transplanting or injecting the hematopoietic stem cell prepared by the method of claim 1 into an individual other than a human.

11. A humanized animal model produced by the method of constructing a humanized animal model of claim 10.
